(19) Europäisches Patentamt
European Patent Office
Office européen des brevets

(11) Publication number: **0 349 963 A2**

(12) **EUROPEAN PATENT APPLICATION**

(21) Application number: 89112136.0

(22) Date of filing: 03.07.89

(51) Int. Cl.⁴: **C07C 43/17 , C07C 41/24**

(30) Priority: 04.07.88 IT 2121188

(43) Date of publication of application: 10.01.90 Bulletin 90/02

(84) Designated Contracting States: BE DE ES FR GB NL SE

(71) Applicant: AUSIMONT S.r.l.
31, Foro Buonaparte
I-20100 Milano(IT)

(72) Inventor: Gervasutti, Claudio
2/4, Via P. Sarpi
I-30172 Mestre Venezia(IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40(DE)

(54) **Process for preparing perfluoro-(2-bromoethylvinylether).**

(57) Process for preparing perfluoro-(2-bromoethylvinylether) of formula
$BrF_2C-CF_2-O-CF=CF_2$
by dechlorination of 2-bromo-1′,2′-dichloroperfluorodiethylether of formula
$BrF_2C-CF_2-O-CClF-CClF_2$
in the presence of copper and in a solvent of formula

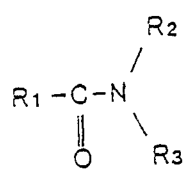

wherein
$R_1$ and $R_2$ are H, $CH_3$ or $C_2H_5$ and
$R_3$ is $CH_3$ or $C_2H_5$;
preferably at temperatures of from 100 to 160 °C.

EP 0 349 963 A2

# PROCESS FOR PREPARING PERFLUORO-(2-BROMOETHYLVINYLETHER)

The present invention relates to the preparation of perfluoro-(2-bromoethylvinylether) (PFBEVE), which is of considerable interest from a commercial point of view and is, for example, used as comonomer in the preparation of fluoropolymers.

From the relevant technical literature several methods for preparing perfluoro-(2-bromoethylvinylether) are known.

One of the suggested methods (EP-A-79 555) consists in reacting tetrafluoroethylene and bromodifluoroacetylfluoride in the presence of KF and iodine in order to obtain 1-bromo-1,1,2,2,4,4,5,5-octafluoro-5-iodo-3-oxapentane of formula

$CF_2ICF_2OCF_2CF_2Br$

said iodo-ether then being subjected to a treatment with Zn and a solvent, in order to eliminate IF according to the reaction:

$$ICF_2CF_2OCF_2CF_2Br \xrightarrow[\text{solvent}]{\text{Zn}} CF_2{=}CFOCF_2CF_2Br + IF$$

However, the above final reaction step is not illustrated by any specific example and the yield of end product is not reported.

In US-A-4 340 750 a process is disclosed for preparing fluorovinylethers of formula

$XR_fCF_2OCF=CF_2$

wherein X stands for several groups, among others bromine, and $R_f$ represents a bifunctional $(C_{1-20})$-perfluoroalkyl group.

In this case, the final reaction step:

$XR_fCF_2OCF_2CF_2I \rightarrow XR_fCF_2OCF=CF_2$

involves the presence of a catalyst selected from a large number of metals such as Mg, Cu, Zn, Zn-Cu, Zn-Cd, Zn-Pd, Zn-Hg, Sn and Sb and of an inert, apolar, aprotic solvent such as benzonitrile, α-naphthonitrile, diphenylether and their mixtures.

In the above US patent no specific examples in which X is Br and $R_f$ is $CF_2$ and in which Cu and an aliphatic amide are used are reported.

Another method for preparing PFBEVE comprises the dechlorination of 2-bromo-1′,2′-dichloroperfluorodiethylether

$BrF_2C\text{-}CF_2\text{-}O\text{-}CClF\text{-}CClF_2$.

A method for preparing the last-mentioned compound is disclosed in applicant's IT-A-22 348 A/86 and comprises the reaction of perfluorobromoethyl hypofluorite of formula

$CBrF_2CF_2OF$

with the olefin of formula

$CFCl=CFCl$

at a temperature of from -40 to -150 °C.

The dechlorination of the resulting ether according to the reaction:

$$BrF_2C\text{-}CF_2\text{-}O\text{-}CClF\text{-}CClF_2 \xrightarrow[\text{solvent}]{\text{metal}} BrF_2C\text{-}CF_2\text{-}O\text{-}CF{=}CF_2$$

is a particular β-elimination which only affects the two adjacent chlorine atoms, the third halogen (Br) not being involved.

It is not easy to cause the above reaction to proceed selectively. In fact, dependent on the particular pair metal/solvent used, the course of the reaction may be very different from the one outlined above or it may even happen that no reaction at all occurs (even after long reaction times). The reaction may also result in various products according to the following reaction schemes:

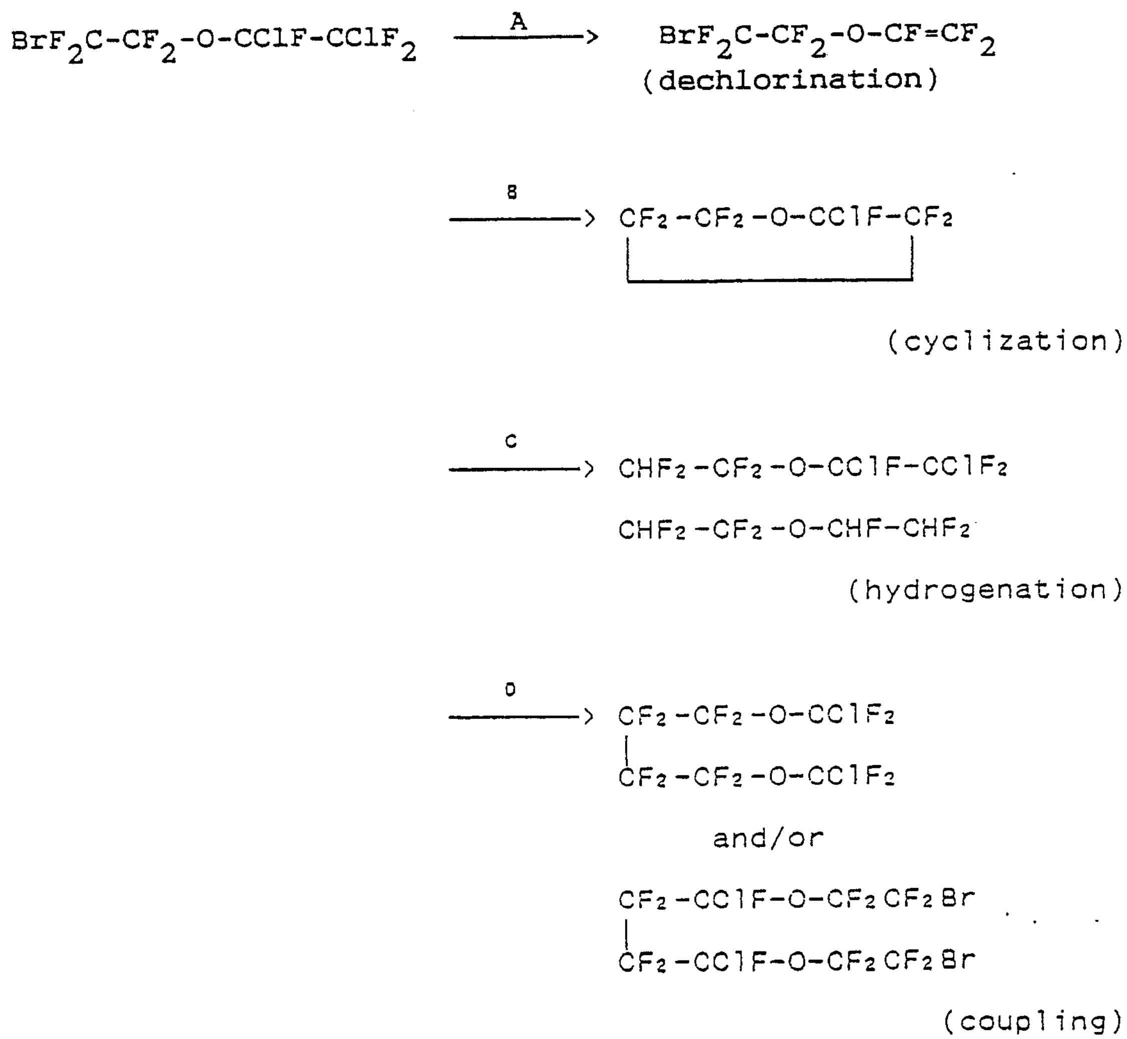


Furthermore, the desired product according to the above scheme (A) in its turn can undergo hydrogenation with the byproduct of formula
$CHF_2CF_2O\text{-}CF=CF_2$
being formed.

Metals such as Zn, conventionally used in dehalogenation reactions proved to be scarcely suitable for the above specific reaction (step "A").

It has now, surprisingly, been found that the dechlorination of
$BrF_2C\text{-}CF_2\text{-}O\text{-}CClF\text{-}CClF_2$
takes place in the presence of Cu when an aprotic solvent is used which consists of an aliphatic amide of formula

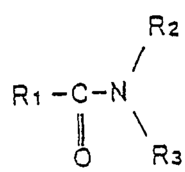

wherein $R_1$ and $R_2$, the same or different from each other, are H or $(C_1\text{-}C_2)$-alkyl groups and
$R_3$ is a $(C_1\text{-}C_2)$-alkyl group.

Therefore, the object of the present invention is a process for preparing PFBEVE, comprising the dehalogenation of a compound of formula
$BrF_2C\text{-}CF_2\text{-}O\text{-}CClF\text{-}CClF_2$
in the presence of metallic Cu and of an apolar aprotic solvent selected from the above aliphatic amides.

Preferably, the solvent is used under anhydrous conditions.

In fact, it was found that, when aliphatic amides containing more than 500 ppm of water are used, the above undesired products (step "C") may be formed, with a consequent reduction of the yield of desired product.

Therefore, the anhydrous conditions with respect to solvent and reaction medium determine the purity of the PFBEVE obtained once the above secondary reactions are prevented from taking place by using the suitable metal/solvent pair.

Among the aliphatic amides suitable for the purpose of the present invention momomethylformamide, dimethylformamide, momoethylformamide, diethylformamide, dimethylacetamide and dimethylpropionamide may be cited. Mixtures of said amides can also be used. In any case, dimethylformamide and dimethylacetamide are particularly preferred.

The selectivity of the above reaction is even more surprising if it is taken into account that the use of alkali metal salts such as NaI or NaF and $K_2CO_3$, conventionally used as catalyst and buffer for free acidity in dechlorination reactions of perfluoroalkylvinylethers, proved to be harmful, blocking the dechlorination reaction completely.

Several metal/solvent pairs were tested which were scarcely effective in the dechlorination reaction according to the present invention.

In fact, when protic solvents (alcohols) were used, independently of the metal a very high yield of the hydrogenated product was obtained and the desired bromoethylvinylether was completely absent.

Similarly, when aprotic solvents different from the aliphatic amides defined above were used, an extremely low conversion and a very low reactivity were observed. Furthermore, dimethylformamide, according to the present invention effective in the presence of copper, proved to be an unsuitable solvent when it was used with other metals such as Sn, Mg and Zn. In this case, although high conversions were obtained, the selectivity with respect to PFBEVE was low owing to the formation of large amounts of hydrogenated products.

Although, for the purposes of the present invention any type of commercial copper of any size can be used, electrolytic copper in the form of a very fine powder is preferred.

The reaction temperature is a function of the boiling point of the reaction mixture. When DMFA is used, said temperature ranges from about 100 to about 160 °C and preferably is in the range of from 130 to 150 °C.

The amount of solvent used in the reaction medium is not critical. At any rate, a sufficient homogenization of the catalyst powder and of the reactant mass must be ensured.

The molar ratio of Cu to the ether to be dechlorinated usually ranges form about 0.25 : 1 to about 4 : 1, preferably from about 0.5 : 1 to about 3 : 1 and particularly from about 1 : 1 to about 2.5 : 1.

The reaction time usually ranges from 40 up to 60 minutes and is not a critical parameter of the present invention.

The process according to the present invention makes it possible to prepare perfluoro-(2-bromoethylvinylether) in very high yields. As already mentioned above, PFBEVE is used as comonomer in the preparation of fluoropolymers because of its ability to introduce active sites for vulcanization reactions of the peroxy type.

The following examples are given in order to illustrate the present invention without limiting it.

## EXAMPLE 1

A 250 ml glass reactor was charged with 50 ml of DMFA with low water content (40 ppm) and 11 g of an extremely fine powder of electrolytic copper were gradually added under stirring. After homogenization of the whole mass, the reaction mixture was heated until a temperature of 80 to 100 °C was reached inside the reactor.

At this point, 30 g of compound of formula

$BrF_2C-CF_2-O-CClF-CClF_2$

dissolved in additional 50 ml of DMFA were added. After an induction period of about 45 to 60 minutes and at a reactor temperature of 135 °C vapours of PFBEVE began to be released and, after having been condensed by an overhead condenser, were subjected to gas-chromatographic analysis.

25 g of a mixture were obtained which, after having been washed with water in order to remove dimethylformamide, according to the gas-chromatographic analysis showed the following molar composition:

| | |
|---|---|
| $BrF_2C-CF_2-O-CF=CF_2$ | 65 % |
| $HF_2C-CF_2-O-CClF-CClF_2$ | 0.5 % |
| unreacted $BrF_2C-CF_2-O-CClF-CClF_2$ | 34.5 % |

The conversion was, thus, 65.5% by mol and the selectivity to perfluoro-(2-bromoethylvinylether) was higher than 98% by mol.

## EXAMPLE 2

The procedure of example 1 was repeated, but using dimethylformamide containing approximately 0.6% of water.

At the end of the reaction, after a washing with water, 20 g of an organic mixture which, according to the gas-chromatographic analysis, showed the following molar composition, were recovered:

| | |
|---|---|
| $BrF_2C-CF_2-O-CF=CF_2$ | 20% |
| $HF_2C-CF_2-O-CClF-CClF_2$ | 38% |
| unreacted $BrF_2C-CF_2-O-CClF-CClF_2$ | 40% |

Accordingly, the conversion was 60% by mol and the selectivity was about 35% by mol.

## EXAMPLE 3

In the reactor used in the preceding tests dimethylformamide having a water content of approximately 500 ppm was placed.

The reaction temperature was kept constant at 145° C.

At the end of the reaction, after a washing with water, an organic mixture which, according to the gas-chromatographic analysis showed the following molar composition, was recovered:

| | |
|---|---|
| $BrF_2C-CF_2-O-CF=CF_2$ | 50% |
| $HF_2C-CF_2-O-CClF-CClF$ | 9% |
| unreacted $BrF_2C-CF_2-O-CClF-CClF_2$ | 40% |

The conversion of the starting product was 60% and the selectivity to PFBEVE was higher than 85%.

## EXAMPLE 4

A 250 ml glass reactor was charged with 30 ml of N,N-dimethylacetamide and 7.5 g of an extremely fine powder of electrolytic copper were gradually added under stirring. After homogenization of the whole mass,the reaction mixture was heated until a temperature of 80 to 100° C was reached inside the reactor.

At this point, 23 g of compound of formula

$BrF_2C-CF_2-O-CClF-CClF_2$,

dissolved in additional 25 ml of N,N-dimethylacetamide were slowly added. After an induction period of about 45 to 60 minutes and at a reactor temperature of 165° C vapours of PFBEVE began to be released and, after having been condensed by an overhead condenser, were subjected to gas-chromatographic analysis.

15g of a mixture were recovered which, after having been washed with water in order to remove N,N-dimethylacetamide, according to gas-chromatographic analysis showed the following molar composition:

| | |
|---|---|
| $BrF_2C-CF_2-O-CF=CF_2$ | 20 % |
| $HF_2C-CF_2-O-CClF-CClF_2$ | 1.5 % |
| $BrF_2C-CF_2-O-CClF-CClF_2$ | 78.5 % |

Thus, the conversion was 21.5% and the selectivity to perfluoro-(2-bromoethylvinylether) was about 93% by mol.

Example 5 (Comparative Example)

The procedure of example 1 was repeated, but using 18 g of a powder of electrolytic Cu and 5 g of $K_2CO_3$, dried at 120 °C for 15 hours, dissolved in 120 ml of dimethylformamide having a low water content (40 ppm).

Then 50 g of $BrF_2C-CF_2-O-CClF-CClF_2$, dissolved in 50 ml of DMFA, were slowly added thereto. An organic mixture was recovered which, according to gas-chromatography,showed the following molar composition:

| | |
|---|---|
| $BrF_2C-CF_2-O-CF=CF_2$ | 1 % |
| $HF_2C-CF_2-O-CClF-CClF_2$ | 4 % |
| unreacted $BrF_2-CF_2-O-CClF-CClF_2$ | 95 % |

Example 6 (Comparative Example)

The procedure of example 1 was repeated, but using 2.5 g of a powder of electrolytic Cu and 5 g of NaI, dried at 120 °C for 15 hours, dissolved in 10 ml of anhydrous dimethylformamide.

Then 10 g of $BrF_2C-CF_2-O-CClF-CClF_2$, dissolved in 10 ml of DMFA, were slowly added thereto.

7 g of an organic mixture were recovered which, according to gas-chromatography, had the following molar composition:

| | |
|---|---|
| $BrF_2C-CF_2-O-CF=CF_2$ | 1 % |
| $HF_2C-CF_2-O-CClF-CClF_2$ | 2 % |
| unreacted $BrF_2C-CF_2-O-CClF-CClF_2$ | 97 % |

Accordingly, the conversion was 3%.

Example 7 (Comparative Example)

The procedure of example 1 was repeated, but using 4.5 g of a powder of electrolytic Cu and 1 g of NaF, dried at 120 °C for 15 hours, dissolved in 25 ml of anhydrous dimethylformamide.

20 g of $BrF_2C-CF_2-O-CClF-CClF_2$, dissolved in 25 ml of DMFA, were slowly added thereto.

18 g of an organic mixture were recovered which, according to gas-chromatography, showed the following molar composition:

| | |
|---|---|
| $BrF_2C-CF_2-O-CF=CF_2$ | 1.2 % |
| $HF_2C-CF_2-O-CClF-CClF_2$ | 0.8 % |
| unreacted $BrF_2C-CF_2-O-CClF-CClF_2$ | 98 % |

The conversion was, thus, 2%.

Example 8 (Comparative Example)

By following the procedure of the preceding examples, several metal/solvent pairs were tested in order to determine the resulting selectivity to PFBEVE.

The characteristics of the reaction and the results obtained are reported in the following table.

TABLE

| Metal/Solvent Pair | Metal/Reactant Molar Ratios | Temperature | Conversion, % by mol | Products |
|---|---|---|---|---|
| Zn/DMFA | 1.3 | 150° | 50 | 3% PFBEVE |
| Mg/DMFA | 1.3 | 150° | 20 | Byproducts |
| Sn/DMFA | 1.7 | 150° | 50 | Byproducts |
| Pt/DMFA | 1.0 | 150° | -- | -- |
| Zn/DMSO | 1.1 | 100° | 10 | Byproducts |
| Sn/dioxane | 1.5 | 100° | 5 | Byproducts |
| Cu/dioxane | 1.8 | 100° | 4 | Byproducts |
| Cu/acrylonitrile | 2.0 | 80° | -- | -- |
| Cu/cyclohexane | 2.0 | 160° | -- | -- |

## Claims

1. Process for preparing perfluoro(2-bromoethylvinylether),comprising the dechlorination of 2-bromo-1′,2′-dichloro-perfluorodiethylether in the presence of copper metal as catalyst and of an aprotic solvent of formula

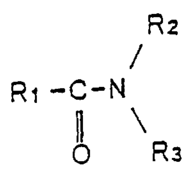

wherein
$R_1$ and $R_2$, the same or different from each other, are H or ($C_1$-$C_2$)-alkyl groups and
$R_3$ is a ($C_1$-$C_2$)-alkyl group.

2. Process according to claim 1, wherein the water content of the aprotic solvent is not higher than 500 ppm and preferably is equal to or less than about 40 ppm.

3. Process according to any one of claims 1 and 2, wherein the reaction temperature ranges from about 100° C to about 160° C.

4. Process according to any one of claims 1 to 3, wherein the copper metal is electrolytic copper.

5. Process according to any one of claims 1 to 4, wherein the copper metal is used in the form of a fine powder.

6. Process according to any one of claims 1 to 5, wherein the aprotic solvent is selected from monomethylformamide, dimethylformamide, monoethylformamide, diethylformamide, dimethylacetamide,dimethylpropionamide, and mixtures thereof.

7. Process according to any one of claims 1 to 6, wherein the aprotic solvent is dimethylformamide and/or dimethylacetamide.